# EUROPEAN PATENT APPLICATION

(11) **EP 3 549 780 A2**
(43) Date of publication of application: **09.10.2019**
(21) Application number: 19158522.3
(22) Date of filing: 21.02.2019
(51) Int. Cl.: B41M 5/52

(54) **COATING SOLUTION FOR FORMING INK RECEIVING LAYER AND METHOD OF PRODUCING COATING SOLUTION FOR FORMING INK RECEIVING LAYER**

(30) Priority: 12.03.2018 JP 2018044009
(71) Applicant: Funai Electric Co., Ltd., Daito, Osaka, 574-0013 (JP)
(72) Inventor: EDAKA, Keiichi, Lexington, KY 40511 (US); MIYAZAKI, Yuki, Osaka 574-0013 (JP)
(74) Representative: Becker Kurig Straus

(57) **Abstract**

To provide a coating solution for forming an ink receiving layer through which it is possible to form an ink receiving layer using the coating solution for forming an ink receiving layer having little effect on a living body, it is possible to prevent a drying time from being too long, and it is possible to prevent a burden on a print target living body from increasing. The coating solution for forming a receiving layer contains water, alcohol including at least one of ethanol and isopropyl alcohol, and a binder agent containing a water-soluble polymer, in which the alcohol content is an amount of 35 weight% or more and 90 weight% or less, and is applied to a living body.

## Description

### BACKGROUND

### Technical Field

The disclosure relates to a coating solution for forming an ink receiving layer and a method of producing a coating solution for forming an ink receiving layer.

### Description of Related Art

In the related art, a printer that performs printing on a medium on which an ink receiving layer is provided is known (for example, refer to Patent Document 1 (Japanese Laid-Open No. 2002-361936)).

Patent Document 1 discloses an inkjet printer that performs printing on a nail attachment component on which a water-based ink image receiving layer (ink receiving layer) is provided by an ink jet head. The nail attachment component is used after being attached to a nail of a person after printing is performed. In the inkjet printer described in Patent Document 1, since the nail attachment component is used after being attached to a nail of a person after printing is performed, before the nail attachment component is attached to a person, a water-based ink image receiving layer (ink receiving layer) is applied to the nail attachment component in advance, and printing is performed.

However, unlike Patent Document 1, when printing is directly performed on a living body such as a nail of a person, in order to form an ink receiving layer, it is necessary to use a coating solution for forming an ink receiving layer having little effect on a living body (human body). In addition, when printing is directly performed on a living body such as a nail of a person, if a drying time of a coating solution for forming an ink receiving layer is long, since a burden on a living body on which printing is performed becomes higher, it is then desirable to shorten the drying time.

### SUMMARY

The disclosure had been made to solve the above problems. The disclosure provides a coating solution for forming an ink receiving layer and a method of producing a coating solution for forming an ink receiving layer through which it is possible to form an ink receiving layer using a coating solution for forming an ink receiving layer having little effect on a living body, it is possible to prevent a drying time from being too long, and it is possible to prevent a burden on a print target living body from being too high.

A coating solution for forming an ink receiving layer according to the disclosure contains water, alcohol including at least one of ethanol and isopropyl alcohol, and a binder agent containing a water-soluble polymer, in which the alcohol content is an amount of 35 weight% or more and 90 weight% or less, and is applied to a living body.

In the coating solution for forming an ink receiving layer according to an embodiment of the disclosure, when the configuration as described above is used, since ethanol and isopropyl alcohol have little effect on a living body compared to other alcohols, it is possible to form an ink receiving layer using a coating solution for forming an ink receiving layer having little effect on a living body. In addition, compared to when a content of alcohol is less than 35 weight%, it is possible to prevent a drying time from being too long. Here, when a content of alcohol is larger, the binder agent is unlikely to dissolve, and the viscosity is higher. That is, compared to when a content of alcohol is more than 90 weight%, the binder agent can dissolve and it is possible to prevent the viscosity from becoming too large. As a result, it is possible to form an ink receiving layer using the coating solution for forming an ink receiving layer having little effect on a living body, it is possible to prevent a drying time from being too long, and it is possible to prevent a burden on a print target living body from increasing.

In the coating solution for forming an ink receiving layer according to an embodiment, preferably, the alcohol content is an amount of more than 50 weight%. In such a configuration, since it is then possible to increase a proportion of alcohol that is readily volatilized, it is possible to effectively shorten a drying time.

In the coating solution for forming an ink receiving layer according to an embodiment, preferably, the binder agent contains polyvinyl alcohol. In such a configuration, it is possible to effectively reduce an effect of the coating solution for forming a receiving layer on a living body.

In this case, preferably, the polyvinyl alcohol in the binder agent has a degree of polymerization of 1,000 or more and a degree of saponification of 60 mol% or more. In such a configuration, compared to when a degree of polymerization of a polyvinyl alcohol is less than 1,000 and a degree of saponification is less than 60 mol%, it is possible to increase the mechanical strength of the receiving layer and increase the water resistance. Accordingly, it is possible to prevent cracks and peeling off of the receiving layer.

In the coating solution for forming an ink receiving layer according to an embodiment, preferably, a fluorescent agent is also included. In such a configuration, since when irradiated with excitation light such as ultraviolet radiation, the coating solution for forming an ink receiving layer emits light, it is possible to easily check a range in which the coating solution for forming an ink receiving layer has been applied.

Preferably, the coating solution for forming an ink receiving layer according to an embodiment is used for a precoating agent for a nail printer. In such a configuration, when printing is directly performed on a nail by a nail printer, it is possible to prevent a burden on a human body from increasing.

A method of producing a coating solution for forming an ink receiving layer according to an embodiment of the disclosure includes mixing and dissolving a binder agent containing a water-soluble polymer into and in a solvent containing water and alcohol including at least one of ethanol and isopropyl alcohol; and then additionally adding alcohol to produce a solution in which there is 35 weight% or more and 90 weight% or less of the alcohol.

In the method of producing a coating solution for forming an ink receiving layer according to an embodiment of the disclosure, when the configuration as described above is used, since ethanol and isopropyl alcohol have little effect on a living body compared to other alcohols, it is possible to form an ink receiving layer using a coating solution for forming an ink receiving layer having little effect on a living body. In addition, compared to when a content of alcohol is less than 35 weight%, it is possible to prevent a drying time from being too long. Here, when a content of alcohol is larger, the binder agent is unlikely to dissolve, and the viscosity is higher. That is, compared to when a content of alcohol is more than 90 weight%, the binder agent can dissolve and it is possible to prevent the viscosity from becoming too large. As a result, it is possible to provide a method of producing a coating solution for forming an ink receiving layer through which it is possible to form an ink receiving layer using a coating solution for forming an ink receiving layer having little effect on a living body, it is possible to prevent a drying time from being too long, and it is possible to prevent a burden on a print target living body from increasing. In addition, the binder agent is mixed into and dissolved in a solvent containing water and alcohol and alcohol is then additionally added. Therefore, it is possible to dissolve the coating solution for forming an ink receiving layer without any of the binder agent failing to dissolve or precipitating. Here, the inventors conducted extensive studies and as a result, found that there is an effect that it is possible for the binder agent to dissolve in the coating solution for forming an ink receiving layer with an alcohol content of 35 weight% or more and 90 weight% or less without any of the binder agent according to this production method failing to dissolve or precipitating.

In the method of producing a coating solution for forming an ink receiving layer according to an embodiment, preferably, when the binder agent is mixed into and dissolved in the solvent, the binder agent is mixed into and dissolved in a solvent in which there is a greater weight of water than of alcohol. In such a configuration, compared to a solvent in which there is a greater weight of alcohol than of water, the binder agent can easily dissolve in the solvent.

In the method of producing a coating solution for forming an ink receiving layer according to an embodiment, preferably, when a binder agent is mixed into and dissolved in a solvent, the binder agent is mixed into the solvent, stirred and heated, and thus the binder agent dissolves in the solvent. In such a configuration, the binder agent can reliably dissolve in the solvent by stirring and heating.

In the method of producing a coating solution for forming an ink receiving layer according to an embodiment, preferably, when a binder agent is mixed into and dissolved in a solvent, the binder agent is mixed into the solvent a plurality of times in a divided manner. In such a configuration, since it is possible to prevent the binder agent from forming lumps in the liquid, the binder agent can easily dissolve in the solvent.

In the method of producing a coating solution for forming an ink receiving layer according to an embodiment, preferably, the binder agent has completely dissolved in the solvent, and the alcohol is then added thereto. In such a configuration, after the binder agent dissolves in the solvent, even if alcohol is additionally added, since the binder agent does not precipitate and the viscosity does not become excessively larger, it is possible to easily increase a content of the alcohol. This effect was found as a result of extensive studies conducted by the inventors.

In the method of producing a coating solution for forming an ink receiving layer according to an embodiment, preferably, the binder agent dissolves in the solvent, and the alcohol is then added dropwise or added by spraying. In such a configuration, since it is possible to prevent the occurrence of a part in which a concentration of alcohol locally increases due to the alcohol added by dropwise addition or spraying of the alcohol, it is possible to effectively prevent the binder agent from precipitating.

In a configuration in which the binder agent dissolves in the solvent and alcohol is then added, preferably, the alcohol added after the binder agent dissolves in the solvent has a greater weight than the alcohol contained in the solvent. In such a configuration, since the binder agent can dissolve in a solvent having a small amount of alcohol, the binder agent can easily dissolve. In addition, since an amount of alcohol added can then be increased, it is possible to increase an amount of alcohol. Accordingly, it is possible to effectively shorten a drying time of the coating solution for forming an ink receiving layer.

In a configuration in which the binder agent dissolves in the solvent and alcohol is then added, preferably, the binder agent dissolves in the solvent, and the alcohol is then added with stirring without heating. In such a configuration, since the binder agent does not precipitate and the alcohol dissolves without heating after the binder agent dissolves, it is possible to prevent evaporation of the alcohol due to heating and effectively increase a content of the alcohol.

In the method of producing a coating solution for forming an ink receiving layer according to an embodiment, preferably, the binder agent contains polyvinyl alcohol. In such a configuration, it is possible to effectively reduce an effect of the coating solution for forming a receiving layer on a living body.

In this case, preferably, the polyvinyl alcohol in the binder agent has a degree of polymerization of 1,000 or more and a degree of saponification of 60 mol% or more. In such a configuration, compared to when a degree of polymerization of a polyvinyl alcohol is less than 1,000 and a degree of saponification is less than 60 mol%, it is possible to increase the mechanical strength of the receiving layer and increase the water resistance. Accordingly, it is possible to prevent cracks and peeling off of the receiving layer.

In the method of producing a coating solution for forming an ink receiving layer according to the second aspect, preferably, a fluorescent agent is additionally added. In such a configuration, since when irradiated with excitation light such as ultraviolet radiation, the coating solution for forming an ink receiving layer emits light, it is possible to easily check a range in which the coating solution for forming an ink receiving layer has been applied.

In this case, preferably, the binder agent dissolves in the solvent, and a fluorescent agent is then added. In such a configuration, when a fluorescent agent is colored, unlike the case in which a fluorescent agent is added before the binder agent dissolves in the solvent, since the solvent is not colored, dissolution of the binder agent can be easily confirmed visually.

According to the disclosure, it is possible to form an ink receiving layer using a coating solution for forming an ink receiving layer having little effect on a living body, it is possible to prevent a drying time from being too long, and it is possible to prevent a burden on a print target living body from increasing.

### BRIEF DESCRIPTION OF THE DRAWINGS

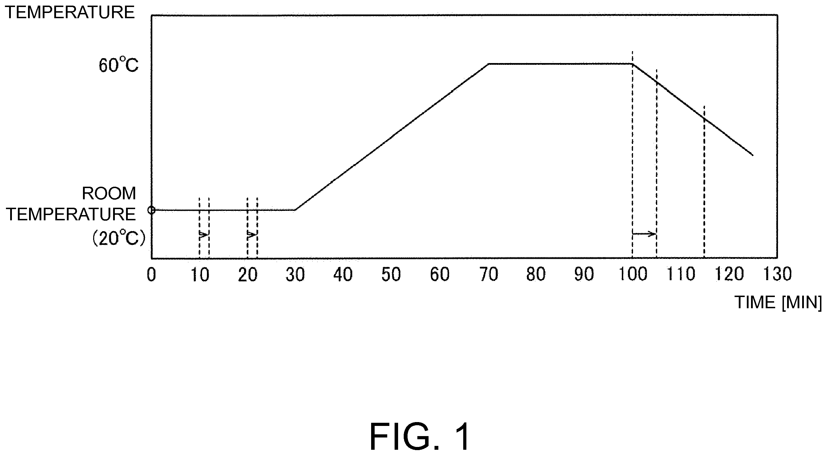

FIG. 1 is a diagram for explaining a method of producing a coating solution for forming an ink receiving layer according to an embodiment.

### DESCRIPTION OF THE EMBODIMENTS

Specific embodiments of the disclosure will be described below with reference to the drawings.

A coating solution for forming an ink receiving layer of the present embodiment is used to form an ink receiving layer when printing is performed by a printer. Specifically, the coating solution for forming an ink receiving layer is applied to a printing area on which an underlayer is coated. That is, an ink receiving layer is formed so that an ink is easily taken into the printing area. The printing area is set on a living body. The printing area is, for example, a nail or the skin of a person. Therefore, the coating solution for forming an ink receiving layer needs to be composed of components having little effect on a living body.

Printing of an ink on a printing area in which an ink receiving layer is formed is performed by an inkjet printer. In the inkjet printer, a water-based ink is used. The ink receiving layer is formed to fix a water-based ink to the printing area. The coating solution for forming an ink receiving layer is used for, for example, a precoating agent for a nail printer.

Here, in the present embodiment, the coating solution for forming an ink receiving layer contains water, alcohol including at least one of ethanol and isopropyl alcohol, and a binder agent containing a water-soluble polymer, in which the alcohol content is an amount of 35 weight% or more and 90 weight% or less, and is applied to a living body. Preferably, in the coating solution for forming an ink receiving layer, the alcohol is contained in an amount of more than 50 weight%. More preferably, in the coating solution for forming an ink receiving layer, the alcohol is contained in an amount of more than 50 weight% and less than 60 weight%.

In addition, the binder agent in the coating solution for forming an ink receiving layer contains polyvinyl alcohol (PVA). In addition, preferably, the polyvinyl alcohol in the binder agent has a degree of polymerization of 1,000 to 2,000. Here, the degree of polymerization of a polyvinyl alcohol is an average degree of polymerization of polymers contained. More preferably, the polyvinyl alcohol in the binder agent has a degree of polymerization of 1,500 or more. Most preferably, the polyvinyl alcohol in the binder agent has a degree of polymerization of 1,700 or more.

In addition, preferably, the polyvinyl alcohol in the binder agent has a degree of saponification of 60 mol% or more. Here, a degree of saponification of the polyvinyl alcohol is an average degree of saponification of polymers contained. That is, the polyvinyl alcohol as the binder agent contains a carboxylic acid ester such as an acetic acid ester in addition to hydroxy groups.

In addition, the coating solution for forming an ink receiving layer may further contain a fluorescent agent. The fluorescent agent emits light when excitation light such as ultraviolet rays is emitted. In the coating solution for forming an ink receiving layer, the fluorescent agent is contained in an amount of 0.1 to 3 weight%. Preferably, in the coating solution for forming an ink receiving layer, the fluorescent agent is contained in an amount of less than 1 weight%. More preferably, in the coating solution for forming an ink receiving layer, the fluorescent agent is contained in an amount of about 0.1 weight%.

In addition, the coating solution for forming an ink receiving layer may further contain a surfactant. In the coating solution for forming an ink receiving layer, the surfactant is contained in an amount of about 0.1 to 5 weight%. In addition, in the coating solution for forming an ink receiving layer, the surfactant is contained in an amount of about 3 weight%. Accordingly, it is possible to improve adhesion of the coating solution for forming an ink receiving layer to a coating area (ink printing area). Surfactants include, for example, a cationic surfactant. Cationic surfactants include, for example, Polyquaternium-7. Preferably, in the coating solution for forming an ink receiving layer, the cationic surfactant is contained in an amount of about 0.1 weight% to 5 weight%. More preferably, in the coating solution for forming an ink receiving layer, the cationic surfactant is contained in an amount of about 0.5 weight% to 2 weight%.

In addition, the coating solution for forming an ink receiving layer has a viscosity of about 200 mPa·s to 1500 mPa·s at room temperature. Preferably, the coating solution for forming an ink receiving layer has a viscosity of about 400 mPa·s to 900 mPa·s at room temperature. Accordingly, the coating solution for forming an ink receiving layer can be easily applied.

Next, a method of producing a coating solution for forming an ink receiving layer will be described with reference to FIG. 1.

Here, in the present embodiment, a method of producing a coating solution for forming an ink receiving layer includes mixing and dissolving a binder agent (polyvinyl alcohol) containing a water-soluble polymer into and in a solvent containing water and alcohol including at least one of ethanol and isopropyl alcohol, and then additionally adding alcohol to produce a solution in which there is 35 weight% or more and 90 weight% or less of the alcohol. Specifically, when the binder agent is mixed into and dissolved in the solvent, the binder agent is mixed into and dissolved in a solvent in which there is a greater weight of water than of alcohol.

In addition, when a binder agent is mixed into and dissolved in a solvent, the binder agent is mixed into the solvent, stirred and heated, and thus the binder agent dissolves in the solvent. In addition, when a binder agent is mixed into and dissolved in a solvent, the binder agent is mixed into the solvent a plurality of times in a divided manner. In addition, the binder agent has completely dissolved in the solvent, and the alcohol is then added thereto.

In addition, the binder agent dissolves in the solvent, and the alcohol is then added dropwise or added by spraying. In addition, the alcohol added after the binder agent dissolves in the solvent has a greater weight than the alcohol contained in the solvent. In addition, the binder agent dissolves in the solvent, and the alcohol is then added with stirring without heating. In addition, the binder agent dissolves in the solvent, and a fluorescent agent is then added.

According to an example of the method of producing a coating solution for forming an ink receiving layer, a coating solution for forming an ink receiving layer is produced by mixing water:ethanol (alcohol):polyvinyl alcohol (binder agent): fluorescent agent: surfactant in proportions of 34.4:56:6.5:0.1:3.

At a time point of 0 minutes in FIG. 1, a low concentration ethanol solution is added into a reaction chamber. The low concentration ethanol solution is a solution in which 20 weight% of a total amount of ethanol (alcohol) added is mixed into a total amount of deionized water (water) added. In this example, water:ethanol=34.4:11.2. Stirring is performed, and at a time point of 10 minutes, a part of polyvinyl alcohol (binder agent) is added to the low concentration ethanol solution (solvent). For a predetermined time from the time point of 10 minutes, 50 weight% of a total amount of polyvinyl alcohol added is incorporated thereinto. At this time point, mixing proportions (weights) of the solution are water:ethanol:polyvinyl alcohol=34.4:11.2:3.25. Polyvinyl alcohol is incorporated into the solvent over a predetermined time with stirring.

Stirring is performed, and for a predetermined time from the time point of 20 minutes, the remaining part of polyvinyl alcohol is added to the low concentration ethanol solution (solvent). At a time point of 20 minutes, 50 weight% of a total amount of polyvinyl alcohol added is incorporated thereinto. At this time point, mixing proportions (weights) of the solution are water:ethanol:polyvinyl alcohol=34.4:11.2:6.5. Polyvinyl alcohol is incorporated into the solvent over a predetermined time with stirring. A dissolution reaction occurs at room temperature (about 20 °C) with stirring from a time point of 0 minutes to one of 30 minutes.

At a time point of 30 minutes, heating is performed first and the temperature of the solution gradually increases. From a time point of 30 minutes to a time point of 70 minutes, the temperature gradually increases, and the solution is heated to about 60 °C. The solution is heated and stirred.

At a time point of 100 minutes, when the polyvinyl alcohol has completely dissolved in the solvent, heating is stopped, and the remaining ethanol is added with stirring. For a predetermined time from the time point of 100 minutes, the remaining ethanol is added to the solution. At a time point of 100 minutes, 80 weight% of a total amount of ethanol added is incorporated thereinto. At this time point, mixing proportions (weights) of the solution are water:ethanol:polyvinyl alcohol=34.4:56:6.5. Ethanol is added dropwise and incorporated thereinto for a predetermined time while the solution is stirred.

As described above, ethanol is added dropwise with stirring while maintaining a high temperature environment, and an ethanol concentration gradually increases. A highly concentrated polyvinyl alcohol solution in this procedure is stable even if the temperature is then returned to room temperature, and does not gel or crystallize again.

At a time point of 115 minutes, the fluorescent agent and the surfactant are added to the solution. Then, stirring is continued, and at a time point of 125 minutes, stirring is terminated, and production of the coating solution for forming an ink receiving layer is completed. As the fluorescent agent, a fluorescent agent dispersion liquid in which a fluorescent pigment has dispersed in water using AS (acrylonitrile styrene copolymer (styrene-acrylonitrile copolymer)) and an anionic surfactant (sodium butyl naphthalene sulfonate) in advance in order to prevent sedimentation of a fluorescent pigment is used. 0.1% shown in the above mixing proportion is a weight proportion of only a pigment.

Here, when the receptivity of an ink is supplemented by adding a cationic surfactant using polyvinyl alcohol with a relatively low degree of polymerization, significant sedimentation is observed when leaving at room temperature for about one week. On the other hand, no sedimentation occurs in the coating solution for forming an ink receiving layer of the disclosure. That is, in the related art, in order to prevent different types of surfactant in a solution from causing unintended bonding, attention needs to be paid to selection of a dispersant and a surfactant used in a dispersion liquid. On the other hand, in the coating solution for forming an ink receiving layer of the disclosure, any type of dispersion liquid may be used.

### (Effects of embodiment)

In the present embodiment, the following effects can be obtained.

In the present embodiment, ethanol and isopropyl alcohol are used as alcohols. Accordingly, since alcohol having little effect on a living body compared to other alcohols can be used, it is possible to form an ink receiving layer using a coating solution for forming an ink receiving layer having little effect on a living body. In addition, in the coating solution for forming an ink receiving layer, the alcohol content is an amount of 35 weight% or more and 90 weight% or less. Accordingly, compared to when a content of alcohol is less than 35 weight%, it is possible to prevent a drying time from being too long. Here, when a content of alcohol is larger, the binder agent is unlikely to dissolve, and the viscosity is higher. That is, compared to when a content of alcohol is more than 90 weight%, the binder agent can dissolve and it is possible to prevent the viscosity from becoming too large. As a result, it is possible to form an ink receiving layer using the coating solution for forming an ink receiving layer having little effect on a living body, it is possible to prevent a drying time from being too long, and it is possible to prevent a burden on a print target living body from increasing.

In addition, in the present embodiment, the alcohol content is an amount of more than 50 weight%. Accordingly, since it is possible to increase a proportion of alcohol that is readily volatilized, it is possible to effectively shorten a drying time.

In addition, in the present embodiment, the binder agent contains polyvinyl alcohol. Accordingly, it is possible to effectively reduce an effect of the coating solution for forming a receiving layer on a living body.

In addition, in the present embodiment, the polyvinyl alcohol in the binder agent has a degree of polymerization of 1,000 or more and a degree of saponification of 60 mol% or more. Accordingly, compared to when a degree of polymerization of a polyvinyl alcohol is less than 1,000 and a degree of saponification is less than 60 mol%, it is possible to increase the mechanical strength of the receiving layer and increase the water resistance. As a result, it is possible to prevent cracks and peeling off of the receiving layer. In addition, in the present embodiment, the polyvinyl alcohol in the binder agent has a degree of polymerization of 2,000 or less. The higher the degree of polymerization, the higher the viscosity will be. When the viscosity is too high, it is difficult to use the coating solution for forming an ink receiving layer. The coating solution for forming an ink receiving layer is difficult to apply, unevenness (irregularities) easily forms on the surface of the ink receiving layer, and the ink receiving layer becomes thick and the drying time becomes long.

In addition, in the present embodiment, a fluorescent agent is also included. Accordingly, since when irradiated with excitation light such as ultraviolet radiation, the coating solution for forming an ink receiving layer emits light, it is possible to easily check a range in which the coating solution for forming an ink receiving layer has been applied.

In addition, in the present embodiment, the coating solution is used for a precoating agent for a nail printer. Accordingly, when printing is performed on a nail by a nail printer, it is possible to prevent a burden on a human body from increasing.

In addition, in the present embodiment, a binder agent containing a water-soluble polymer is mixed into and dissolved in a solvent containing water and alcohol including at least one of ethanol and isopropyl alcohol, and alcohol is then additionally added to produce a solution in which there is 35 weight% or more and 90 weight% or less of the alcohol. Accordingly, the binder agent is mixed into and dissolved in a solvent containing water and alcohol and alcohol is then additionally added. Therefore, it is possible to dissolve the coating solution for forming an ink receiving layer without any of the binder agent failing to dissolve or precipitating.

In addition, in the present embodiment, when the binder agent is mixed into and dissolved in the solvent, the binder agent is mixed into and dissolved in a solvent in which there is a greater weight of water than of alcohol. Accordingly, compared to a solvent in which there is a greater weight of alcohol than of water, the binder agent can easily dissolve in the solvent.

In addition, in the present embodiment, when a binder agent is mixed into and dissolved in a solvent, the binder agent is mixed into the solvent, stirred and heated, and thus the binder agent dissolves in the solvent. Accordingly, the binder agent can reliably dissolve in the solvent by stirring and heating.

In addition, in the present embodiment, when a binder agent is mixed into and dissolved in a solvent, the binder agent is mixed into the solvent a plurality of times in a divided manner. Accordingly, since it is possible to prevent the binder agent from forming lumps in the liquid, the binder agent can easily dissolve in the solvent.

In addition, in the present embodiment, the binder agent has completely dissolved in the solvent, and the alcohol is then added thereto. Accordingly, after the binder agent dissolves in the solvent, even if alcohol is additionally added, since the binder agent does not precipitate and the viscosity does not become excessively larger, it is possible to easily increase a content of the alcohol.

In addition, in the present embodiment, the binder agent dissolves in the solvent, and the alcohol is then added dropwise or added by spraying. Accordingly, since it is possible to prevent the occurrence of a part in which a concentration of alcohol locally increases due to the alcohol added by dropwise addition or spraying of the alcohol, it is possible to effectively prevent the binder agent from precipitating.

In addition, in the present embodiment, the alcohol added after the binder agent dissolves in the solvent has a greater weight than the alcohol contained in the solvent. Accordingly, since the binder agent can dissolve in a solvent having a small amount of alcohol, the binder agent can easily dissolve. In addition, since an amount of alcohol added can then be increased, it is possible to increase an amount of alcohol. As a result, it is possible to effectively shorten a drying time of the coating solution for forming an ink receiving layer.

In addition, in the present embodiment, the binder agent dissolves in the solvent, and the alcohol is then added with stirring without heating. Accordingly, since the binder agent does not precipitate and the alcohol dissolves without heating after the binder agent dissolves, it is possible to prevent evaporation of the alcohol due to heating and effectively increase a content of the alcohol.

### (Examples)

Next, experiments performed in order to confirm effects of the above embodiment will be described.

Experiments were performed while changing an amount of ethanol and an amount of water. Here, components other than ethanol and water were the same.

When an amount of ethanol was 0 weight% and an amount of water was 93 weight% (Comparative Example 1), a drying time of the coating solution for forming an ink receiving layer was 10 minutes. When an amount of ethanol was 20 weight% and an amount of water was 73 weight% (Comparative Example 2), a drying time of the coating solution for forming an ink receiving layer was 8 minutes. When an amount of ethanol was 35 weight% and an amount of water was 58 weight% (Example 1), a drying time of the coating solution for forming an ink receiving layer was 7 minutes and 30 seconds. When an amount of ethanol was 58 weight% and an amount of water was 35 weight% (Example 2), a drying time of the coating solution for forming an ink receiving layer was 6 minutes. That is, when 35 weight% or more and 90 weight% or less of alcohol was contained, it is possible to effectively shorten a drying time.

Next, experiments were performed while a degree of polymerization of a polyvinyl alcohol was changed.

When a degree of polymerization of a polyvinyl alcohol was 600 (Comparative Example 3), if printing was performed by a printer, bleeding increased. In this case, it was difficult to use it for a receiving layer for printing. On the other hand, when a degree of polymerization of a polyvinyl alcohol respectively were 1,000 and 1,700 (Example 3, 4), if printing was performed by a printer, there was no bleeding, and a favorable state was obtained. That is, when a degree of polymerization of a polyvinyl alcohol was 1,000 or more, it was possible to increase the mechanical strength of the receiving layer and increase water resistance.

Explaining a relationship between a concentration of ethanol and a drying time is shown in table 1. Explaining a relationship between a degree of polymerization of a polyvinyl alcohol and print quality is shown in table 2.

**Table 1**

| AMOUNT OF ETHANOL | AMOUNT OF WATER | DRYING TIME |
|---|---|---|
| 0% | 93% | 10 MINUTES |
| 20% | 73% | 8 MINUTES |
| 35% | 58% | 7 MINUTES AND 30 SECONDS |
| 58% | 35% | 6 MINUTES |

**Table 2**

| DEGREE OF POLYMERIZATION OF PVA | PRINT QUALITY |
|---|---|
| 600 | LARGE BLEEDING |
| 1000 | FAVORABLE |
| 1700 | FAVORABLE |

### (Modified examples)

Here, the embodiments and examples disclosed herein are only examples in all respects and should not be considered as restrictive. The scope of the disclosure is defined not by the description of the embodiments and examples described above, but by the scope of the claims and furthermore, includes meanings equivalent to the scope of the claims and all modifications (modified examples) within the scope of the claims.

For example, while an example in which the coating solution for forming an ink receiving layer is applied to a human body has been exemplified in the above embodiment, the disclosure is not limited thereto. In the disclosure, the coating solution for forming an ink receiving layer may be applied to a living body other than a human body. For example, the coating solution may be applied to animals and plants. In addition, the coating solution for forming an ink receiving layer may be applied to a part other than a living body. For example, the coating solution may be applied to leather, vinyl, or plastic (for example, polycarbonate). The coating solution for forming an ink receiving layer of the disclosure can also reduce an effect on a material other than the above living body.

In addition, while an example in which the coating solution for forming an ink receiving layer is applied onto an underlayer has been exemplified in the above embodiment, the disclosure is not limited thereto. In the disclosure, the coating solution for forming an ink receiving layer may be directly applied to a living body without an underlayer.

In addition, while a configuration in which the coating solution for forming an ink receiving layer contains ethanol as alcohol has been exemplified in the above embodiment, the disclosure is not limited thereto. In the disclosure, the coating solution for forming an ink receiving layer may contain isopropyl alcohol as alcohol. In addition, the alcohol may be a mixture of ethanol and isopropyl alcohol.

In addition, while an example in which the coating solution for forming an ink receiving layer is used for forming a receiving layer when printing is performed by a printer has been exemplified in the above embodiment, the disclosure is not limited thereto. In the disclosure, when an ink is applied to a living body using a pen or the like, a receiving layer may be formed using the coating solution for forming an ink receiving layer.

In addition, while an example in which the binder agent dissolves in the solvent, and alcohol is then added with stirring without heating has been exemplified in the above embodiment, the disclosure is not limited thereto. In the disclosure, the binder agent may be dissolved in the solvent, and the alcohol may be then added with stirring and also heating.

## Claims

1. A coating solution for forming an ink receiving layer **characterized by** comprising:
water;
an alcohol comprising at least one of ethanol and isopropyl alcohol; and
a binder agent containing a water-soluble polymer,
wherein the alcohol is contained in an amount of 35 weight% or more and 90 weight% or less.

2. The coating solution for forming an ink receiving layer according to claim 1, wherein the binder agent contains polyvinyl alcohol.

3. The coating solution for forming an ink receiving layer according to claim 1 or 2, wherein the polyvinyl alcohol in the binder agent has a degree of polymerization of 1,000 or more and a degree of saponification of 60 mol% or more.

4. The coating solution for forming an ink receiving layer according to any one of claims 1 to 3, further comprising a fluorescent agent.

5. A method of producing a coating solution for forming an ink receiving layer **characterized by** comprising:
mixing and dissolving a binder agent containing a water-soluble polymer into and in a solvent containing water and an alcohol comprising at least one of ethanol and isopropyl alcohol; and
then additionally adding the alcohol to produce a solution in which there is 35 weight% or more and 90 weight% or less of the alcohol.

6. The method of producing a coating solution for forming an ink receiving layer according to claim 5, wherein, when the binder agent is mixed into and dissolved in the solvent, the binder agent is mixed into and dissolved in the solvent in which there is a greater weight of water than of the alcohol.

7. The method of producing a coating solution for forming an ink receiving layer according to claim 5 or 6, wherein the binder agent is mixed into the solvent, stirred and heated, and thus the binder agent dissolves in the solvent, when the binder agent is mixed into and dissolved in the solvent.

8. The method of producing a coating solution for forming an ink receiving layer according to any one of claims 5 to 7, wherein the binder agent is mixed into the solvent a plurality of times in a divided manner, when the binder agent is mixed into and dissolved in the solvent.

9. The method of producing a coating solution for forming an ink receiving layer according to any one of claims 5 to 8, wherein the binder agent dissolves in the solvent and the alcohol is then added dropwise or added by spraying.

10. The method of producing a coating solution for forming an ink receiving layer according to claim 8 or 9, wherein the alcohol added after the binder agent dissolves in the solvent has a greater weight than the alcohol contained in the solvent.

11. The method of producing a coating solution for forming an ink receiving layer according to any one of claims 8 to 10, wherein the binder agent dissolves in the solvent and the alcohol is then added with stirring without heating.

12. The method of producing a coating solution for forming an ink receiving layer according to any one of claims 5 to 11, wherein the binder agent contains a polyvinyl alcohol.

13. The method of producing a coating solution for forming an ink receiving layer according to claim 12, wherein the polyvinyl alcohol in the binder agent has a degree of polymerization of 1,000 or more and a degree of saponification of 60 mol% or more.

14. The method of producing a coating solution for forming an ink receiving layer according to any one of claims 5 to 13, further comprising: additionally adding a fluorescent agent.

15. The method of producing a coating solution for forming an ink receiving layer according to claim 14, wherein the binder agent dissolves in the solvent and the fluorescent agent is then added.
